(19) Europäisches Patentamt
European Patent Office
Office européen des brevets

(11) Numéro de publication: **0 272 981 B1**

(12) # FASCICULE DE BREVET EUROPEEN

(45) Date de publication du fascicule du brevet: **14.11.90**

(51) Int. Cl.⁵: **C07D 313/12**, A61K 31/335

(21) Numéro de dépôt: **87402888.9**

(22) Date de dépôt: **17.12.87**

(54) **Dérivés d'acide dibenzo[be]oxepinne-acétique, leur préparation et leur application en thérapeutique.**

(30) Priorité: **22.12.86 FR 8617990**

(43) Date de publication de la demande:
**29.06.88 Bulletin 88/26**

(45) Mention de la délivrance du brevet:
**14.11.90 Bulletin 90/46**

(84) Etats contractants désignés:
**AT BE CH DE ES FR GB GR IT LI LU NL SE**

(56) Documents cités:
**EP-A- 0 037 254**
**EP-A- 0 198 762**

(73) Titulaire: **SYNTHELABO, 58 rue de la Glacière, F-75013 Paris(FR)**

(72) Inventeur: **Purcell, Thomas A., 47bis, rue de la millière Les Mesnuls, F-78490 Montfort L'Amaury(FR)**
Inventeur: **Rivron, Luc, 5, rue du Château, F-92600 Asnières(FR)**
Inventeur: **Zard, Lydia, 6, Impasse des Quatre vents La Croix Audierne, F-91190 Gif S/Yvette(FR)**

(74) Mandataire: **Ludwig, Jacques et al, SYNTHELABO Service Brevets 22, avenue Galilée, F-92352 LE PLESSIS ROBINSON CEDEX(FR)**

EP 0 272 981 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des dérivés d'acide dibenzo[be]oxepinne-acétique,leur préparation et leur application en thérapeutique.

Les composés de l'invention répondent à la formule (I)

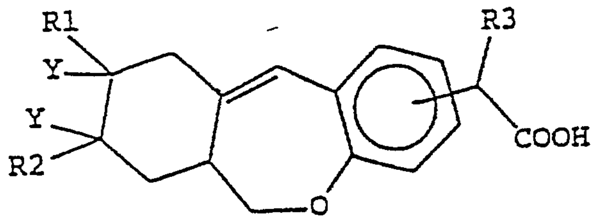

(I)

dans laquelle
R1, R2 et R3, indépendamment l'un de l'autre, représentent chacun un atome d'hydrogène ou un groupe méthyle, et
YY représente une liaison carbon-carbone ou deux atomes d'hydrogène.

Les composés peuvent se présenter à l'état d'acides libres ou des sels qu'ils peuvent former avec des bases acceptables en pharmacologie.

Les diverses formes stéréoisomères que peuvent prendre les composés de formule (I) font également partie de l'invention.

Les composés de l'invention sont utiles comme substances anti-inflammatoires et/ou analgésiques. Des composés de structures analoques, ayant des domaines d'utilistions identiques, sont décrits dans les demandes de breve ts EP 0 198 762.

Conformément à l'invention les composés peuvent être préparés selon le schéma 1 de la page suivante.

Les composés de départ, répondant à la formule (II), dans laquelle YY représente une liaison, sont décrits dans la demande de brevet européen n° 0 198 762.

Si l'on souhaite préparer des composés (I) dans la formule desquels YY représente deux atomes d'hydrogène, on soumet d'abord un ester (II, YY= —) à une hydrogénation sous pression, en présence d'un catalyseur tel que le dioxyde de platine.

SCHEMA 1

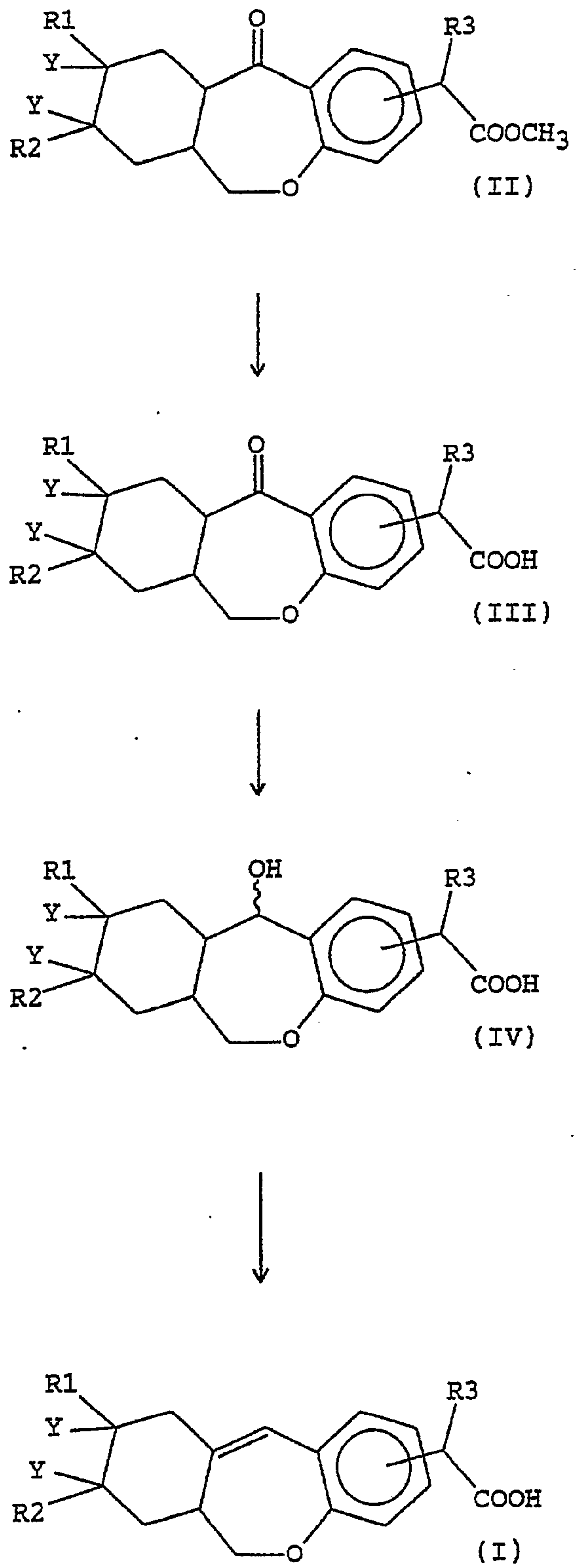

(II)

(III)

(IV)

(I)

On hydrolyse ensuite l'ester de formule (II), par exemple avec de la soude en milieu alcoolique pour obtenir un acide libre de formule (III). Les acides (et leurs esters) de formule (III), dans laquelle YY représente deux atomes d'hydrogène et R1 et R2 représentent deux atomes d'hydrogène, sont également décrits dans la demande de brevet européen n° 0037254.
Selon ce document on les obtient par un procédé illustré par le schéma 2 ci-après.

SCHEMA 2

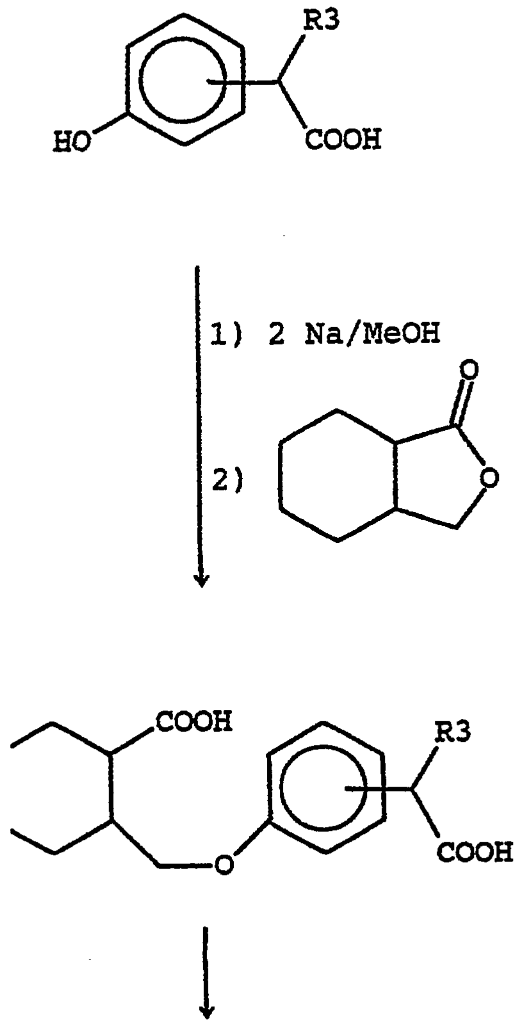


(V)

(VI)

(VII)

(III)

On traite un acide benzèneacétique de formule (V) par le sodium puis on fait réagir le sel ainsi obtenu avec une lactone, l'octahydro isobenzofurannone-1, de formule (VI), ce qui donne le dérivé de formule (VII) qu'on soumet à une cyclisation.
Si la lactone de formule (VI) a une structure stéréochimique déterminée, on aboutit à un intermédiaire de formule (III), et finalement à un composé de formule (I), ayant eux-mêmes des structures stéréochimiques déterminées.
La lactone de formule (VIa) est décrite dans Organic Syntheses (1985), 63, 10 et celle de formule (VIb) peut être synthétisée à partir du diacide de formule (VIc) décrit dans J. Org. Chem. (1963), 28, 48, en passant par l'anhydride correspondant, selon des méthodes analogues à celles décrites dans J. Org. Chem, (1964), 29, 3154 et J. Org. Chem. (1970), 35, 3574.

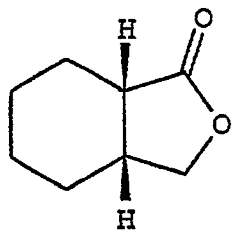

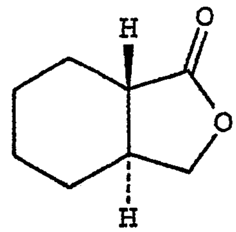

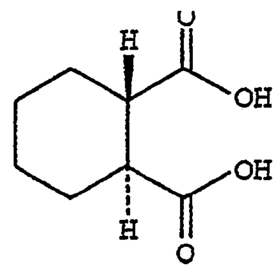

(VIa) (VIb) (VIc)

On réduit ensuite la fonction cétone du composé (III) en fonction alcool secondaire au moyen d'un borohydrure alcalin. Finalement on déshydrate l'alcool (IV) en présence d'acide paratoluènesulfonique, à chaud et dans un solvent tel que le benzène.

Enfin on peut préparer un composé de formule (I) où R3 représente un groupe méthyle à partir d'un composé de formule (I) où R3 représente l'hydrogène en protégeant préalablement ce dernier par estérification au moyen d'un alcool inférieur, par exemple le méthanol, à la température du reflux, puis en alkylant l'ester ainsi obtenu au moyen d'un halogénure tel que l'iodométhane, en présence de diisopropylamidure de lithium (préparé in situ à l'aide de diisopropylamine et de n-butyllithium), dans un solvant tel que le tétrahydrofuranne, puis en repassant à l'acid par hydrolyse de l'ester ainsi obtenu, par exemple avec de la soude aqueuse, à chaud et en milieu alcoolique.

Les exemples suivants illustrent la préparation de quelques conposés conformes à l'invention.
Les microanalyses et les spectres IR et RMN confirment la structure des produits obtenus.

Exemple 1. Acide hexahydro-6,6a,7,8,9,10 dibenzo[be]oxépinne-3-acétique.

a) Oxo-11 octahydro-6,6a,7,8,9,10,10a,11 dibenzo[be]oxépinne-3-acétate de méthyle.
Dans une fiole à hydrogéner de 500 ml on introduit 8,58 g (0,03 mole) de oxo-11 hexahydro-6,6a,7,10,10a,11 dibenzo[be]oxépinne-3-acétate de méthyle, 1 g d'oxyde de platine et 200 ml d'éthanol, et on effectue une hydrogénation sous environ 0,28 MPa pendant 45 minutes.
On sépare le catalyseur par filtration sous argon et on évapore le filtrat, ce qui laisse 8 g d'une huile qu'on utilise telle quelle dans l'étape suivante.

b) Acide oxo-11 octahydro-6,6a,7,8,9,10,10a,11 dibenzo[be]oxépinne-3-acétique.
On traite 8 g de l'huile obtenue selon 1a) à 60°C pendant 1 h par un mélange de 100 ml de méthanol, 100 ml d'eau et 4,5 ml de soude à 30 %. Puis on évapore l'alcool et on extrait les traces d'ester résiduel avec de l'acétate d'éthyle. On acidifie la phase aqueuse avec de l'acide chlorhydrique 1N et on extrait l'acide organique avec de l'acétate d'éthyle.
On sèche la phase organique sur sulfate de magnésium, on la concentre et on la distille entre 185 et 190° sous environ 25 Pa. Après recristallisation du distillat dans l'alcool isopropylique on recueille 6,5 g de solide. F = 99-101°C.

c) Acid hexahydro-6,6a,7,8,9,10 dibenzo[be]oxépinne-3-acétique.
On dissout 5,48 g du solide obtenue selon 1b) dans 100 ml d'éthanol et on ajoute lentement 1,2 g de borohydrure de sodium. On agite pendant 40 minutes puis on concentre le mélange.
On reprend le résidu avec 200 ml d'eau, on ajoute lentement de l'acide chlorhydrique jusqu'à pH = 2, en refroidissant le mélange sur de la grace, puis on l'extrait à l'acétate d'éthyle.
On évapore le solvant à 25°C, et on reprend rapidement le produit, sensible à la chaleur, avec 150 ml de benzène et 0,2 g d'acide paratoluènesulfonique.
On chase l'eau dans un appareil de Dean-Stark en 30 minutes environ, et on concentre le résidu à 40°C sous vide, jusqu'à un volume résiduel d'environ 15 ml, puis on purifie le produit sur colonne de silice en éluant avec un mélange 955 de dichlorométhane/méthanol.
Après recristallisation des fractions pures à froid dans un mélange 8/2 de cyclohexane/éther isopropylique, on recueille 2,5 g du produit pur. F = 145-147°C.

Exemple 2. Acide tétrahydro-6,6a,7,10 dibenzo[be]oxépinne-3-acétique.

a) Acide hydroxy-11 hexahydro 6,6a,7,10,10a,11 dibenzo[be]oxépinne-3-acétique.
Dans un erlenmeyer de 1 l on introduit 2,72 g (0,01 mole) d'acide oxo-11 hexahydro-6,6a,7,10,10a,11 dibenzo[be]oxépinne-3-acétique, 200 ml d'éthanol, et on traite la solution avec 3 g de borohydrure de sodium à 0°C.
Après 30 mn de réaction on évapore le mélange, on reprend le résidu avec de l'eau, on refroidit la suspension dans un bain de grace et on la traite avec de l'acide chlorhydrique jusqu'à pH = 3, pour détruire l'excès de borohydrure de sodium.

On extrait l'alcool obtenu avec de l'acétate d'éthyle. Après séchage sur sulfate de magnésium et évaporation de la phase organique, on recristallise le résidue dans un mélange 8/2 d'acétate d'éthyle/cyclohexane. On obtient 2 g de cristaux. F = 138-140°C.

b) Acide tétrahydro-6,6a,7,10 dibenzo[be]oxépinne-3- acétique.

On soumet 2,7 g d'alcool obtenu selon 2a) à un déshydration dans 300 ml de benzène, au reflux, en présence de 0,1 g d'acide paratoluénesulfonique.

Après 30 mn on évapore le benzène et on purifie le résidu par chromatographie sur colonnne de silice en éluant avec un mélange 9:1 de méthanol/dichlorométhane, et en recristallisant le produit obtenu dans un mélange 7/3 d'éther de pé trole/acétate d'éthyle.

On obtient 1,25 g de cristaux. F = 120-122°C.

Exemple 3. Enantiomère lévogyre de l'acide hexahydro 6,6a,7, 8, 9,10 dibenzo[be]oxépinne-3-acétique.

a) Acide [(Carboxy-2 cyclohexyl)méthoxy]-3 benzèneacétique.

On dissout 1,97 g (0,0856 moles) de sodium dans 50 ml de méthanol anhydre et, sous atmosphère d'argon, on ajoute 6,52 g (0,0428 mole) d'acide hydroxy-3 benzèneacétique. On évapore le solvant et, au sel ainsi obtenu, on ajoute 7,2 g (0,0514 mole) de lactone de formule (VIa). On chauffe le mélange à 195°C pendant 45 mn, on le laisse refroidir, on ajoute de l'eau glacée et de l'acide chlorhydrique à 10 %, et on l'extrait à l'éther. Après séchage sur sulfate de magnésium, décoloration au charbon végétal et évaporation du solvant on obtient 13,64 g de résidu huileux qu'on utilise tel quel dans l'étape suivante.

b) Acide oxo-11 octahydro-6,6a,7,8,9,10,10a,11 dibenzo[be]oxépinne-3-acétique.

On ajoute 120 g d'acide polyphosphorique à 13,5 g de l'huile précédemment obtenue et on chauffe le mélange au bain d'huile à 70°C tout en l'agitant. Dès que cette température est atteinte, on retire le chauffage, car elle se maintient d'elle-même.

La réaction terminée, on verse le mélange dans de l'eau glacée, on l'extrait à l'éther puis au dichlorométhane, on sèche sur sulfate de magnésium et on évapore les deux phases organiques, et on purifie les résidus par chromatographie sur colonne de silice, on réunit les fractions pures et on les distille sous vide. On recueille finalement 1,15 g d'une huile jaune qu'on utilise telle quelle dans l'étape suivante.

c) Acide hydroxy-11 octahydro-6,6a,7,8,9,10,10a,11dibenzo[be]oxépinne-3-acétique.

On dissout 1,15 g de l'huile précédemment obtenue dans 15 ml d'éthanol absolu, on refroidit la solution à -10°C et, sous atmosphère d'argon, on ajoute 0,47 g (0,0125 mole) de borohydrure de sodium. On laisse revenir le mélange à température ambiante et, après 1 h, on le verse dans de l'eau glacée. On évapore l'éthanol, on lave la phase aqeuse à l'éther, on ajoute de l'acide chlorhydrique 2N et on l'extrait à l'éther. Après séchage sur sulfate de magnésium et évaporation sous vide on obtient 1 g d'une huile incolore qu'on utilise telle quelle dans l'étape suivante.

d) Acide hexahydro-6,6a,7,8,9,10 dibenzo[be]oxépinne-3-acétique.

On dissout 1 g de l'huile précédemment obtenue dans 50 ml de benzène anhydre, on ajoute quelques cristaux d'acide paratoluènesulfonique et on chauffe le mélange au reflux en éliminant l'eau avec un appareil de Dean-Stark.

Lorsque toute l'eau est éliminée on évapore le solvant, et on laisse refroidire le mélange qui cristallise.

On sépare 0,6 g d cristaux par filtration, on les recristallise dans le benzène, après les avoir traités au charbon végétal. On obtient finalement 0,27 g d'énantiomère pur.

F = 137-138°C $(\alpha)_D^{20}$ = -28,27° (c = 0,29 ; $CHCl_3$).

Exemple 4. Enaniomère dextrogyre de l'acide hexahydro-6,6a, 7,8,9,1- dibenzo[be]oxépinne-3-acétique.

a) Acide [(carboxy-2 cyclohexyl)méthoxy]-3 benzèneacétique. On dissout 8,2 g (0,355 mole) de sodium dans 100 ml de méthanol anhydre et, sous atmosphère d'argon, on ajoute 27 g (0,18 mole) d'acide hydroxy-3 benzène-acétique.

On évapore le solvant et, au sel ainsi obtenue, on ajoute 30 g (0,214 mole) de lactone de formule (VIb). On chauffe le mélange à 195°C pendant 2 h, on le laisse refroidir, on le verse dans de l'eau glacée, on lave le mélange à l'éther, on ajoute de l'acide chlorhydrique à 10 % à la phase aqueuse et on l'extrait à l'éther.

Après séchage sur sulfate de magnésium et décoloration au charbon végétal, on évapore le solvant et on obtient 48 g de résidu qu'on recristallise dans le toluène. On recueille finalement 22 g de cristaux qu'on utilise tels quels dans l'étape suivante.

b) Acide oxo-11 octahydro-6,6a,7,8,9,10,10a,11 dibenzo[be]oxépinne-3-acétique.

On ajoute 220 g d'acide polyphosphorique à 22 g des cristaux précédemment obtenus et on chauffe le mélange au bain d'huile à 75°C pendant 30 mn.

On le laisse refroidir, on le verse dans de l'eau glacée, on l'extrait à l'éther, on sèche la phase organique sur sulfate de magnésium et on évapore le solvant sous vide. On obtient 15 g de résidue mousseux orangé qu'on utilise tel dans l'étape suivante.

c) Acide hydroxy-11 octahydro-6,6a,7,8,9,10,10a,11 dibenzo[be]oxépinne-3-acétique.

On dissout 15 g (0,0546 mole) du produit précédemment obtenu dans 300 ml d'éthanol absolu, on refroidit

la solution à -10°C et, sous atmosphère d'argon, on ajoute 6,2 g (0,163 mole) de borohydrure de sodium.
On laisse revenir le mélange à température ambiante et, après 2 h, on le verse dans de l'eau glacée. On évapore l'éthanol, on lave la phase aqueuse à l'éther, on ajoute de l'acide chlorhydrique à 10 % et on l'extrait à l'éther.
Après séchage sur sulfate de magnésium et évaporation sous vide on obtient 13 g de résidu mousseux jaune. On les triture dans du dichlorométhane ce qui donne 4,21 g de cristaux qu'on utilise tels quels dans l'étape suivante.

d) Acide hexahydro-6,6a,7,8,9,10 dibenzo[be]oxépinne-3-acétique.

On dissout 4 g (0,0144 mole) des cristaux précédemment obtenus dans 200 ml de benzène anhydre, on ajoute 50 mg d'acide paratoluènesulfonique et on chauffe le mélange au reflux en éliminant l'eau avec un appareil de Dean-Stark.
Après 2 h on concentre la solution limpide obtenue jusqu'à 20 ml environ et on la purifie telle quelle par chromatographie sur collonne de silice en éluant avec un mélange 95/5 de dichlorométhane/méthanol. On récupère 3,17 g de cristaux beiges qu'on recristallise dans le benzène, ce qui donne finalement 2,36 g de cristaux blancs brillants d'énantiomère pur.
F = 136-138°C $(\alpha)_D^{20} = + 29,21$ (c = 0,38 ; $CHCl_3$).

Exemple 5. Acide α-méthyl hexahydro-6,6a,7,8,9,10 dibenzo [be]oxépinne-3-acétique.

a) Hexahydro-6,6a,7,8,9,10 dibenzo[be]oxépinne-3-acétate de méthyle.

On chauffe au reflux un mélange de 5 g (0,0194 mole) d'acide hexahydro-6,6a,7,8,9,10 dibenzo[be]oxépinne-3-acétique et 50 ml de méthanol, additionné de quelques gouttes d'acide sulfurique concentré, pendant 1 h.
On évapore le mélange à sec, on reprend le résidue avec de l'eau, on le neutralise avec du carbonate de sodium et on l'extrait à l'éther.
Après séchange sur sulfate de magnésium et évaporation de l'éther on obtient 4,8 g d'une huile qu'on purifie par chromatographie sur colonne de silice en éluant avec du dichlorométhane. On recueille 4,4 g d'ester qu'on utilise tel quel dans l'étape suivante.

b) α-méthyl hexahydro-6,6a,7,8,9,10 dibenzo[be]oxépinne-3-acétate de méthyle.

On dissout 4,44 ml de diisopropylamine dans 62 ml de tétrahydrofurane, on refroidit la solution à -70°C, et on introduit goutte à goutte 20,5 ml de solution 1,6M de n-butyl-lithium.
On agite le mélange pendant 15 mn et on ajoute, goutte à goutte, 4,3 g (0,0158 mole) de l'ester précédemment obtenu en solution dans 50 ml de tétrahydrofuranne. On agite le mélange pendant 20 mn et, toujours à -70°C, on ajout 2,64 g (0,0186 mole) de iodométhane.
On maintient l'agitation pendant 1 h 30 à -70°C, puis on laisse le mélange revenir à température ambiante, on le verse dans de l'eau glacée, on l'extrait à l'acétate d'éthyle, on sépare la phase organique, on la sèche sur sulfate de magnésium et on l'évapore. On obtient 4 g d'une huile qu'on uti lise telle quelle dans l'étape suivante.

c) Acide α-methyl hexahydro-6,6a,7,8,9,10 dibenzo[be]oxépinne-3-acétique.

On dissout 4 g de l'huile précédemment obtenue dans 25 ml de méthanol, on ajoute de l'eau et 6 ml d'hydroxyde de sodium et on chauffe le mélange pendant 1 h en évaporant le méthanol, puis pendant 2 h au reflux.
On le laisse refroidir, on le lave à l'éther, on acidifie la phase aqueuse, et on extrait le précipité à l'éther. On traite la phase éthérée au charbon végétal, on la sèche sur sulfate de magnésium, on la filtre et on l'évapore. On recristallise le résidu dans le cyclohexane, et on recueille finalement 3 g de produit pur.
F = 143-145°C.

Le tableau de la page suivante illustre les structures et propiétés physique de quelques composés selon l'invention.

Tableau

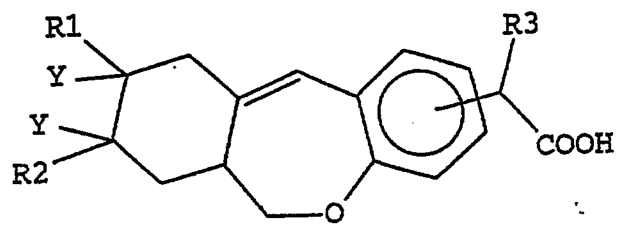

| Composé (Exemple) | Position -CHR3COOH | YY | R1 | R2 | R3 | F(°C) |
|---|---|---|---|---|---|---|
| 1 (1) | 3 | HH | H | H | H | 145-147 |
| (3) | | énantiomère (-) | | | | 137-138 |
| (4) | | énantiomère (+) | | | | 136-138 |
| 2 (2) | 3 | — | H | H | H | 120-122 |
| 3 | 3 | — | $CH_3$ | $CH_3$ | H | 170-171 |
| 4 | 2 | HH | H | H | H | 125-126 |
| 5 (5) | 3 | HH | H | H | $CH_3$ | 143-145 |

Les composés de l'invention ont fait l'objet de divers essais qui ont mis en évidence leur intérêt comme substances à visées thérapeutiques, en particulier anti-inflammatoires.

Leur toxicité aigüe est faible, la dose létale $DL_{50}$ chez la souris étant le plus souvent supérieure à 1000 mg/kg par voie orale.

Pour l'étude de l'activité anti-inflammatoire, des essais ont été effectués sur le test de l'oedème à la carragénine chez le rat selon méthode de Winter et al. ("Carrageenin induced oedema in hind paw of the rat as an assay for anti-inflammatory drugs". Proc. Soc. Exp. Biol. Med. 1962, 111, 544-547). Les animaux utilisés sont des rats CD mâles de Charles River (France) d'un poids moyen de 120 à 130 g, répartis en lots au hasard à l'aide d'une table de répartition.

Les composés sont administrés par voie orale à des doses comprises entre 20 et 200 mg/kg, 1 heure avant l'injection sous l'apronévrose plantaire à l'une des pattes postérieures, de 0,1 ml de carragénine,

en suspension à 1 % dans du sérum physiologique stérile. Les animaux témoins ne reçoivent que le placebo, une solution de Tween 80® à 1 %. l'augmentation du volume de la patte est mesurée 3 heures après l'injection de carragénine au moyen d'un pléthysmomètre Ugo Basile. On détermine graphiquement la dose $DA_{40}$, dose de substance qui diminue de 40 % le volume de l'oedème, par rapport aux animaux témoins. Les $DA_{40}$ des composés de l'invention se situent entre 1 et 50 mg/kg par la voie orale.

L'activité analgésique des composés de l'invention a été montrée dans le test de Koster et al., ("writhing test" à l'acide acétique chez la souris, Fed. Proc., 18, 412, 1959). On administre par voie orale, à des souris $CD_1$ mâles,à jeun depuis 18 heures, le composé à tester en solution dans du Tween 80® à 1%, à raison de 0,2 ml par 20 g de poids corporel ; au bout de 30 mn on administre l'acid acétique (en solution à 0,6 % dans un mélange de carboxyméthylcellulose et de Tween 80®, à raison de 10 ml par kg de poids corporel) par voie intrapéritonéale. Chez les animaux témoins cette injection déclenche en quelques minutes un syndrome d'étirements et de contorsions (writhing) pouvant être considéré comme l'expression d'une douleur abdominale diffuse.
On note le nombre total de contorsions pendant 15 mn, on détermine le pourcentage de protection par rapport au lot témoin, et on calcule la $DA_{50}$ par voie graphique (dose qui protège 50% des animaux).
Les $DA_{50}$ des composés de l'invention se situent entre 75 et 170 mg/kg par la voie orale.

Les résultats des essais montrent que les composés de l'invention peuvent être utilisés comme substances actives de médicaments et compositions pharmaceutiques utilisables pour le traitement d'inflammations et de douleurs d'origines diverses.
A cet effet ils peuvent être présentés sous toutes formes adaptées à l'administration entérale ou parentérale, par exemple sous forme de comprimés, gélules, dragées, sirops, suppositoires, suspensions buvables ou injectables, en association avec des excipients appropriés.
La posologie journalière peut aller de 50 à 1000 mg de substance active.

**Revendications pour les Etats contractants: AT, BE, CH, DE, FR, GB, IT, LI, LU, NL, SE**

1. Composé, sous forme d'isomères optiques purs ou de leurs mélanges, répondant à la formule générale (I)

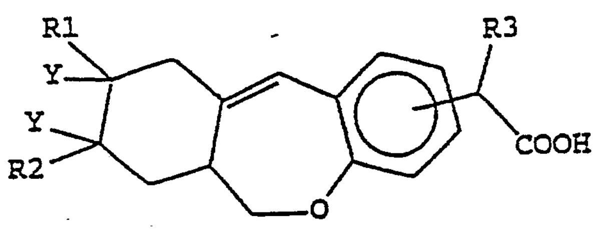

(I)

dans laquelle
R1, R2 et R3, indépendamment l'un de l'autre, représentent chacun un atome d'hydrogène ou un groupe méthyle, et, YY représente une liaison carbone-carbone ou deux atomes d'hydrogène, ainsi que les sels qu'ils peuvent former avec des bases acceptables en pharmacologie.

2. Composé selon la revendication 1, caractérisé en ce que R1, R2 et R3 représentent chacun un atome d'hydrogène, YY représente deux atomes d'hydrgène, et le groupe CH(R3)COOH est en position 3.

3. Procédé de préparation des composés de formule (I) selon la revendication 1, caractérisé en ce que, si l'on souhaite que YY représente deux atomes d'hydrogène, on soumet préalablement un ester de formule (II)

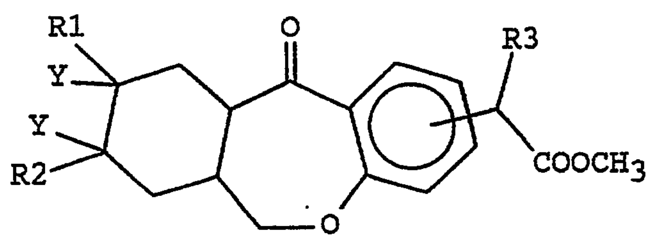

(II)

(dans laquelle R1, R2 et R3 sont tels que définis dans la revendication 1 et YY représente une liaison) à une hydrogénation catalytique pour obtenir un ester de formule (II) où YY représente deux atomes d'hydrogène, puis on hydrolyse l'ester (II) en acide libre de formule (III)

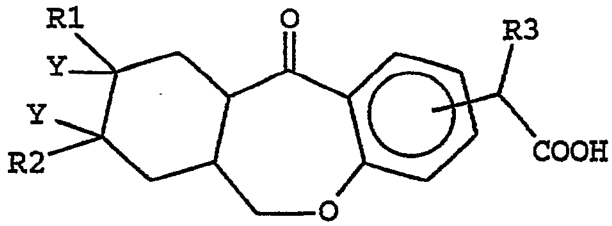

(III)

dont on réduit la fonction cétone en fonction alcool au moyen d'un borohydrure alcalin pour obtenir un alcool de formule (IV)

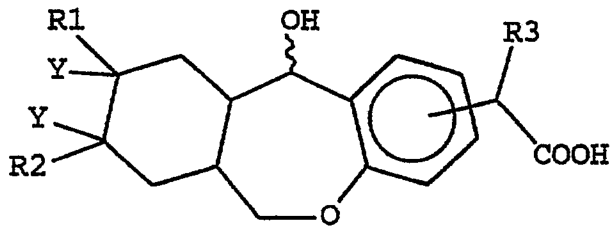

(IV)

puis on déshydrate l'alcool (IV) en présence d'acide paratoluènesulfonique, et, si on le désire, on soumet un composé de formule (I), dans laquelle R3 représente un atope d'hydrogène, à une méthylation pour obtenir un composé de formule (I) dans laquelle R3 représente un groupe méthyle.

4. Procédé selon la revendication 3, caractérisé en ce qu'on prépare un composé de formule (III) dans laquelle YY représente deux atomes d'hydrogène et R1 et R2 représentent deux atomes d'hydrogène, en traitant un acide benzèneacétique de formule (V)

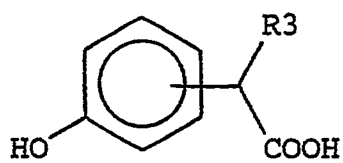

(V)

(dans laquelle R3 est tel que défini dans la revendication 1) par le sodium puis on fait réagir le sel ainsi obtenu avec une lactone de formule (VI)

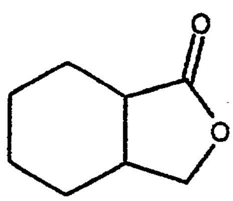

(VI)

pour obtenir un dérivé de formule (VII)

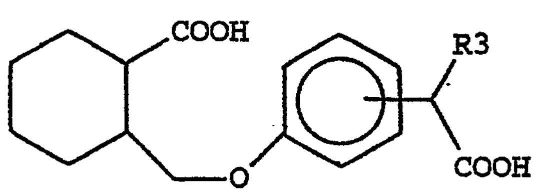

(VII)

et on soumet ce dernier à une cyclisation

5. Médicament caractérisé en ce qu'il est constitué d'un composé selon la revendication 1.

6. Composition pharmaceutique caractérisée en ce qu'elle contient un composé selon la revendication 1, associé à un excipient approprié.

**Revendications pour les Etats contractants: ES, GR**

1. Procédé de préparation des composés de formule générale (I)

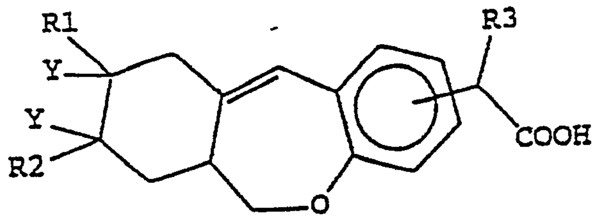

(I)

dans laquelle
R1, R2 et R3, indépendamment l'un de l'autre, représentent chacun un atome d'hydrogène ou un groupe méthyle, et, YY représente une liaison carbone-carbone ou deux atomes d'hydrogène,
procédé caractérisé en ce que, si l'on souhaite que YY représente deux atomes d'hydrogène, on soumet préalablement un ester de formule (II)

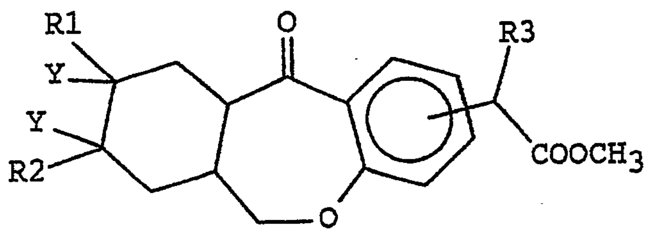

(II)

(dans laquelle R1, R2 et R3 sont tels que définis ci-dessus et YY représente une liaison) à une hydrogénation catalytique pour obtenir un ester de formule (II) où YY représente deux atomes d'hydrogène, puis on hydraulyse l'ester (II) en acide libre de formule (III)

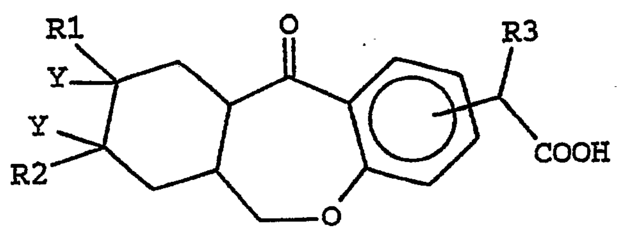

(III)

dont on réduit la fonction cétone en fonction alcool au moyen d'un borohydrure alcalin pour obtenir un alcool de formule (IV)

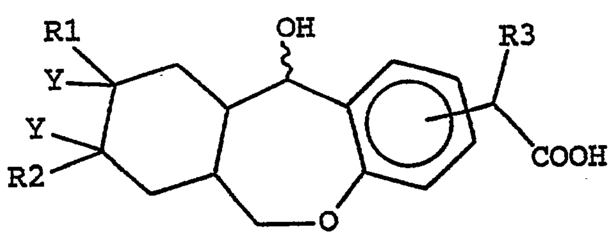

(IV)

puis on déshydrate l'alcool (IV) en présence d'acide paratoluènesulfonique, et, si on le désire, on soumet un composé de formule (I), dans laquelle R3 représente un atome d'hydrogène, à une méthylation pour obtenir un composé de formule (I) dans laquelle R3 représente un groupe méthyle.

2. Procédé selon la revendication 1, caractérisé en ce qu'on prépare un composé de formule (III) dans laquelle YY représente deux atomes d'hydrogène et R1 et R2 représentent deux atomes d'hydrogène, en traitant un acide benzèneacétique de formule (V)

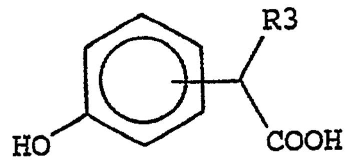

(V)

(dans laquelle R3 est tel que défini dans la revendication 1) par le sodium puis on fait réagir le sel ainsi obtenu avec une lactone de formule (VI)

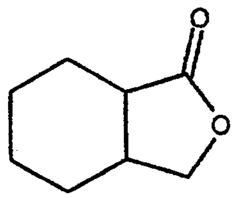

(VI)

pour obtenir un dérivé de formule (VII)

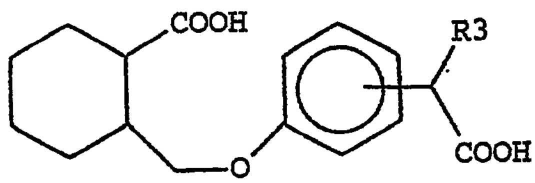

(VII)

et on soumet ce dernier à une cyclisation.

**Claims for the contracting states: AT, BE, CH, DE, FR, GB, IT, LI, LU, NL, SE**

1. Compounds in the form of pure optical isomers or mixtures thereof, corresponding to the general formula (I)

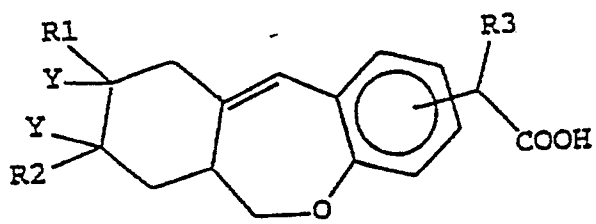

(I)

in which
R1, R2 and R3, independently of one another, each denote a hydrogen atom or a methyl group, and YY denotes a carbon-carbon bond or two hydrogen atoms, as well as the salts which they can form with bases that are acceptable in pharmacology.

2. Compound according to claim 1, characterized in that R1, R2 and R3 each denote a hydrogen atom, YY denotes two hydrogen atoms and the group CH(R3)COOH is at the 3-position.

3. Process for preparing the compounds of formula (I) according to Claim 1, characterized in that , if it is desired that YY shall denote two hydrogen atoms, an ester of formula (II)

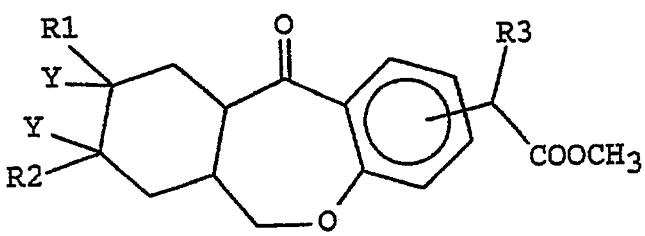

(II)

(in which R1, R2 and R3 are as defined in claim 1 and YY denotes a bond) is subjected beforehand to a catalytic hydrogenation so as to obtain an ester of formula (II) in which YY denotes two hydrogen atoms, the ester (II) is then hydrolysed to the free acid of formula (III)

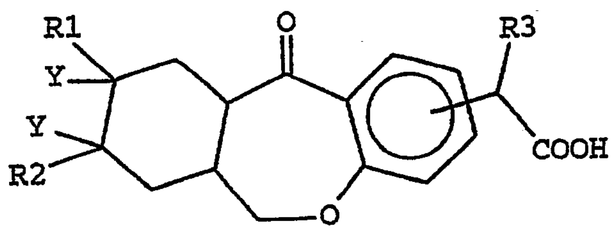

(III)

the ketone group of which is reduced to an alcohol group by means of an alkali metal borohydride to obtain an alcohol of formula (IV)

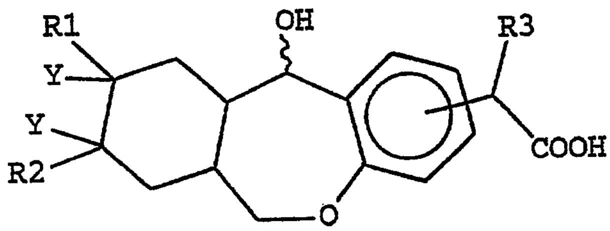

(IV)

the alcohol (IV) is then dehydrated in the presence of para-toluenesulphonic acid and, if so desired, a compound of formula (I) in which R3 denotes a hydrogen atom is subjected to methylation so as to obtain a compound of formula (I) in which R3 denotes a methyl group.

4. Process according to claim 3, characterized in that a compound of formula (III) in which YY denotes two hydrogen atoms and R1 and R2 denote two hydrogen atoms is prepared by treating a benzeneacetic acid of formula (V)

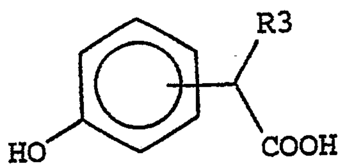

(V)

(in which R3 is as defined in claim 1) with sodium, the salt thereby obtained is then reacted with a lactone of formula (VI)

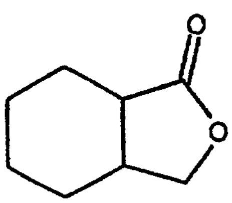

(VI)

to obtain a derivative of formula (VII)

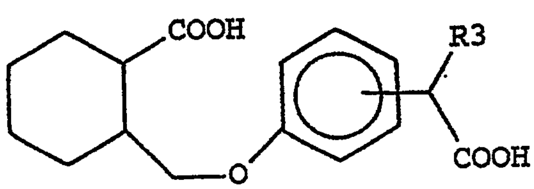

(VII)

and the latter is subjected to a cyclization.

5. Medicinal product, characterized in that it consists of a compound according to claim 1.

6. Pharmaceutical composition, characterized in that it contains a compound according to claim 1, in combination with a suitable excipient.

**Claims for the contracting states: ES, GR**

1. Process for preparing the compounds of general formula (I)

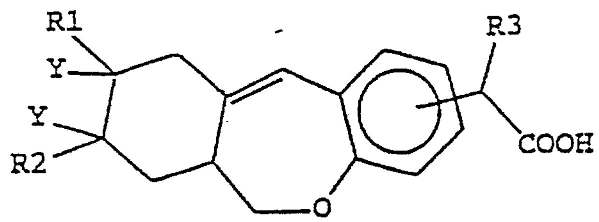

(I)

in which R1, R2 and R3, independently of one another, each denote a hydrogen atom or a methyl group, and YY denotes a carbon-carbon bond or two hydrogen atoms, which process is characterized in that, if it is desired that YY shall denote two hydrogen atoms, an ester of formula (II)

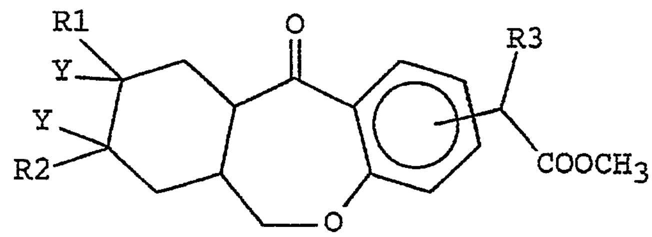

(II)

(in which R1, R2 and R3 are as defined above and YY denotes a bond) is subjected beforehand to a catalytic hydrogenation so as to obtain an ester of formula (II) in which YY denotes two hydrogen atoms, the ester (II) is then hydrolysed to the free acid of formula (III)

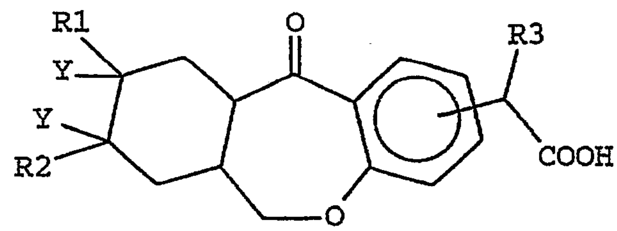

(III)

the ketone group of which is reduced to an alcohol group by means of an alkali metal borohydride to obtain an alcohol of formula (IV)

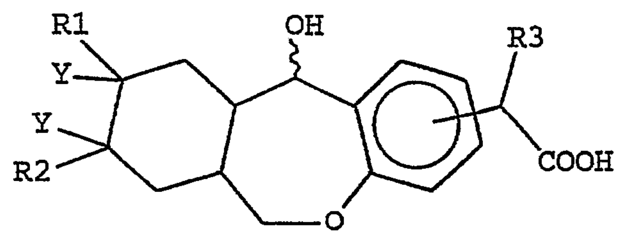

(IV)

the alcohol (IV) is then dehydrated in the presence of para-toluenesulphonic acid and, if so desired, a compound of formula (I) in which R3 denotes a hydrogen atom is subjected to methylation so as to obtain a compound of formula (I) in which R3 denotes a methyl group. 2. Process according to claim 1, characterized in that a compound of formula (III) in which YY denotes two hydrogen atoms and R1 and R2 denote two hydrogen atoms is prepared by treating a benzeneacetic acid of formula (V)

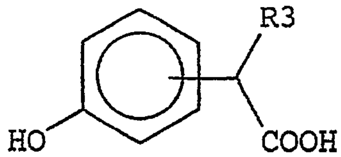

(V)

(in which R3 is as defined in claim 1) with sodium, the salt thereby obtained is then reacted with a lactone of formula (VI)

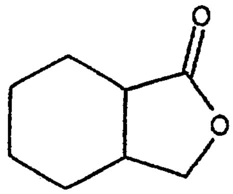

(VI)

to obtain a derivative of formula (VII)

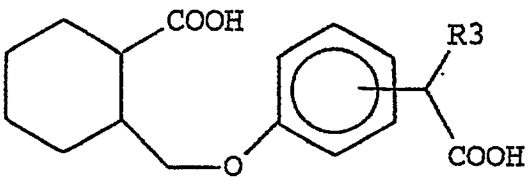

(VII)

and the latter is subjected to a cyclization.

**Patentansprüche für die Vertragsstaaten:** ES, GR

1. Verfahren zur Herstellung von Verbindungen der allgemeinen Formel (I)

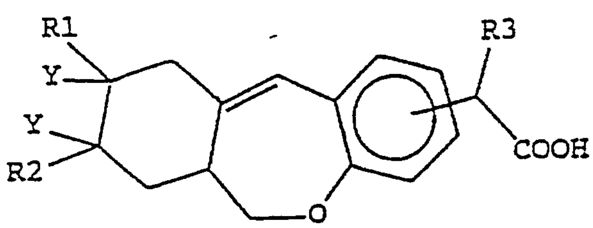

(I)

in welcher R1, R2 und R3 unabhängig voneinander jeweils für ein Wasserstoffatom oder eine Methylgruppe stehen und YY eine Kohlenstoff-Kohlenstoff-Bindung oder zwei Wasserstoffatome darstellen, dadurch gekennzeichnet, dass man, wenn YY für zwei Wasserstoffatome stehen sollen, vorest einen Ester der Formel (II)

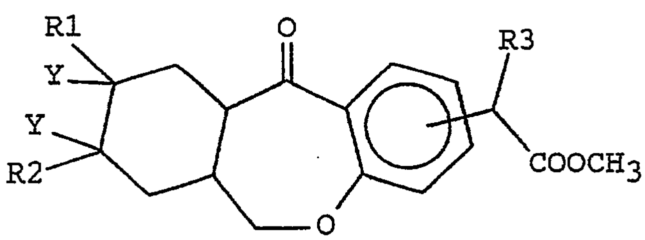

(II)

(in welcher R1, R2 und R3 die oben genannte Bedeutung haben und YY für eine Bindung steht) einer katalytischen Hydrierung unterwirft, um einen Ester der Formel (II) zu erhalten, in dem YY für zwei Wasserstoffatome stehen, dann den Ester (II) zur freien Säure der Formel (III)

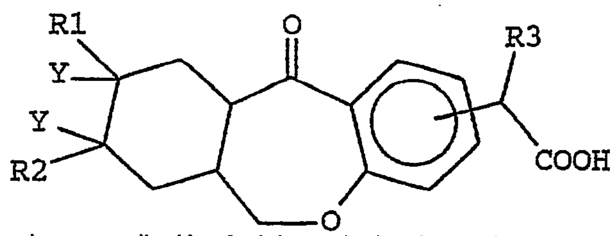

(III)

hydrolysiert, in der man die Ketofunktion mittels eines Alkalibothydrids zur Alkoholfunktion reduziert, um einen Alkohol der Formel (IV)

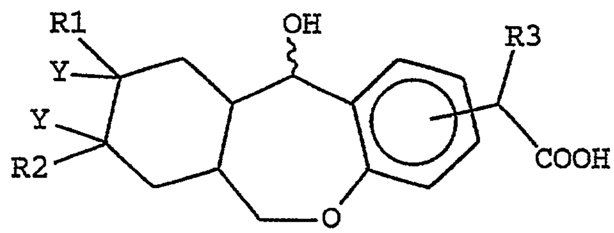

(IV)

zu erhalten, worauf man diesen Alkohol (IV) in Gegenwart von p-Toluolsulfonsäure dehydratisiert und gewünschtenfalls eine Verbindung der Formel (I), in welcher R3 ein Wasserstoffatom bedeutet, einer Methylierung unterwirft, um eine Verbindung der Formel (I) zu erhalten, in welcher R3 eine Methylgruppe darstellt.

2. Verfahren nach Anspruch 1, dadurch gekennzeichnet, dass man eine Verbindung der Formel (III) herstellt, in welcher YY für zwei Wasserstoffatome stehen und R1 und R2 zwei Wasserstoffatome darstellen, indem man eine Benzolessigsäure der Formel (V)

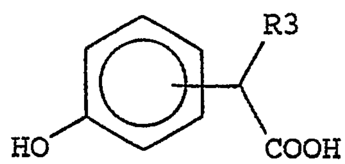

(V)

(in welcher R3 die in Anspruch 1 genannte Bedeutung hat) mit Natrium behandelt und anschliessend das so erhaltene Salz mit einem Lacton der Formel (VI)

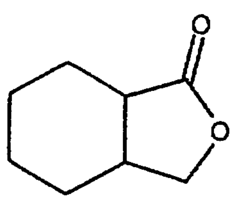

(VI)

reagieren lässt, um ein Derivat der Formel (VII)

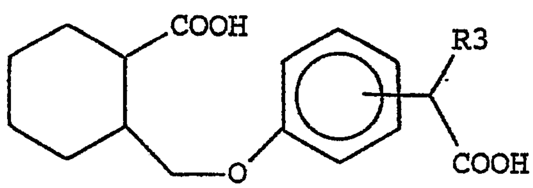

(VII)

zu gewinnen, welch letzteres man einer Zyklisierung unterwirft.

**Patentansprüche für die Vertragsstaaten: AT, BE, CH, DE, FR, GB, IT, LI, LU, NL, SE**

1. Verbindungen in Form der reinen optischen Isomeren oder ihrer Mischungen, die der allgemeinen Formel (I)

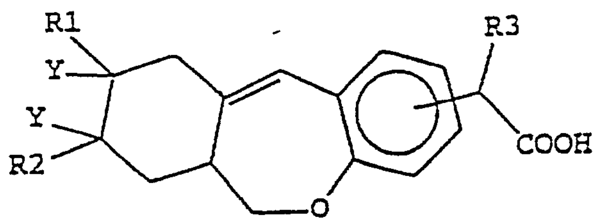

(I)

in welcher R1, R2 und R3 unabhängig voneinander jeweils für ein Wasserstoffatom oder eine Methylgruppe stehen und YY eine Kohlenstoff-Kohlenstoff-Bindung oder zwei Wasserstoffatome darstellen, entsprechen, sowie deren Salze, die sie mit pharmakologisch verwendbaren Basen bilden können.

2. Verbindung nach Anspruch 1, dadurch gekennzeichnet, dass R1, R2 und R3 jeweils für ein Wasserstoffatom stehen, YY zwei Wasserstoffatome darstellen und die Gruppe CH(R3)COOH in 3-Stellung gebunden ist.

3. Verfahren zur Herstellung der Verbindungen der in Anspruch 1 angegebenen Formel (I), dadurch gekennzeichnet, dass man wenn YY für zwei Wasserstoffatome stehen sollen, vorerst einen Ester der Formel (II)

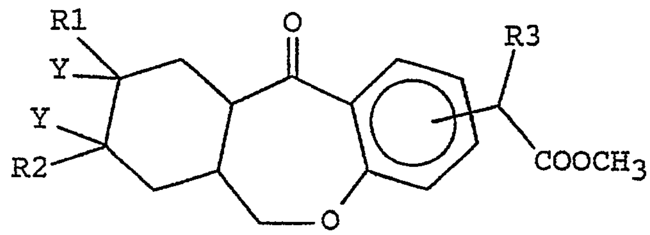

(II)

(in welcher R1, R2 und R3 die in Anspruch 1 angegebene Bedeutung haben und YY für eine Bindung stehen) einer katalytischen Hydrierung unterwirft, um einen Ester der Formel (II) zu erhalten, in dem YY für zwei Wasserstoffatome stehen, dann den Ester (II) zur freien Säure der Formel (III)

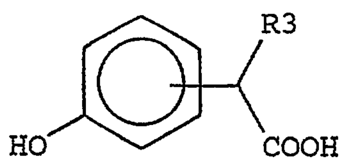

(V)

hydrolysiert, in der man die Ketofunktion mittels eines Alkaliborhydrids zur Alkoholfunktion reduziert, um einen Alkohol der Formel (IV)

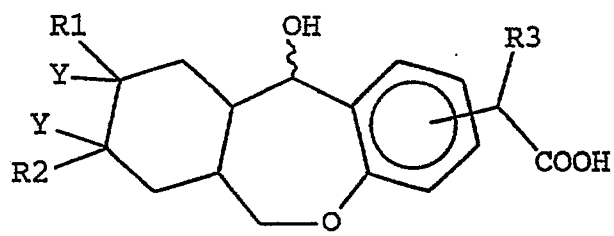

(IV)

zu erhalten, worauf man diesen Alkohol (IV) in Gegenwart von p-Toluolsulfonsäure dehydratisiert und gewünschtenfalls eine Verbindung der Formel (I), in welcher R3 ein Wasserstoffatom bedeutet, einer Methylierung unterwirft, um eine Verbindung der Formel (I) zu erhalten, in welcher R3 eine Methylgruppe darstellt.

4. Verfahren nach Anspruch 3, dadurch gekennzeichnet, dass man eine Verbindung der Formel (III) herstellt, in welcher YY für zwei Wasserstoffatome stehen und R1 und R2 zwei Wasserstoffatome darstellen, indem man eine Benzolessigsäure der Formel (V)

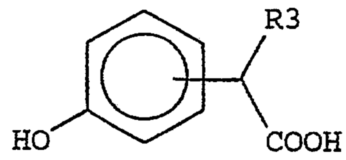

(V)

(in welcher R3 die in Anspruch 1 genannte Bedeutung hat) mit Natrium behandelt und anschliessend das so erhaltene Salz mit einem Lacton der Formel (VI)

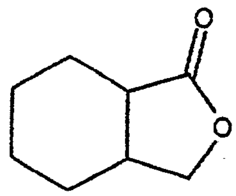

(VI)

reagieren lässt, um ein Derivat der Formel (VII)

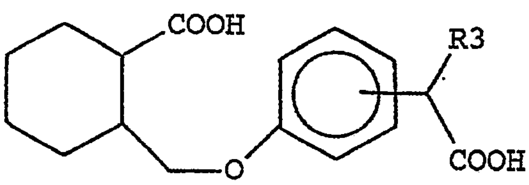

(VII)

zu gewinnen, welch letzteres man einer Zyklisierung unterwirft.

5. Arzneimittel, dadurch gekennzeichnet, dass es aus einer Verbindung gemäss Anspruch 1 besteht.

6. Pharmazeutische Zusammensetzung, dadurch gekennzeichnet, dass sie eine Verbindung nach Anspruch 1 in kombination mit einem geeigneten Trägermaterial enthält.